# NEUE EUROPÄISCHE PATENTSCHRIFT

(11) **EP 0 711 838 B2**
(45) Veröffentlichungstag und Bekanntmachung des Hinweises auf die Entscheidung über den Einspruch: **20.06.2007**
(45) Hinweis auf die Patenterteilung: 17.01.2001
(21) Anmeldenummer: 95116175.1
(22) Anmeldetag: 13.10.1995
(51) Int. Cl.: C12Q 1/56, G01N 33/86

(54) **Verfahren zum spezifischen Nachweis eines aktivierten Gerinnungsfaktors V mit einer erhöhten Stabilität gegenüber aktiviertem Protein C**
Method to specifically detect an activated clotting factor V with an increased stability vis à vis activated protein C
Méthode de détection spécifique d'agent de coagulation V avec une stabilité augmentée vis à vis de la protéine C activée

(30) Priorität: 10.11.1994 DE 4440097; 23.02.1995 DE 19506263
(43) Veröffentlichungstag der Anmeldung: 15.05.1996
(73) Patentinhaber: Dade Behring Marburg GmbH, 35001 Marburg (DE)
(72) Erfinder: Kraus, Michael, Dr., D-35041 Marburg (DE)

(56) Entgegenhaltungen:
- WO-A-93/10261
- WO-A-94/17415
- US-A- 5 051 357
- NATURE, Bd. 369, - 5.Mai 1994 LONDON UK, Seiten 64-67, XP 000563812 R.M. BERTINA ET AL. 'Mutation in blood coagulation factor V associated with resistance to activated protein C.'
- DRUG RESEARCH, Bd. 44, Nr. 6, - 1.Juni 1994 BASEL CH, Seiten 793-797, XP 000562757 H.E. KARGES ET AL. 'Activity of coagulation and fibrinolysis parameters in animals.'
- THROMBOSIS RESEARCH, Bd. 80, Nr. 3, - 1995 NEW YORK NY USA, Seiten 255-264, XP 000564298 M. KRAUS ET AL. 'Coagualtion assay with improved specificity to factor V mutants insensitive to activated protein C.'
- J I Jorquera et al. (1994), The Lancet 344,1162-1163
- X Sun et al. (1994), Blood 83, 3120-3125
- F Ausseil et al. (1994), Proc.Congr. Angio Genève 1994, 61
- L Y Yukelson et al (1991), Toxicon 20, 491-502
- M J P Kahn et al(1972), Thromb.Diath.Haemorrh. 27, 25-32

## Beschreibung

Die Erfindung bezieht sich auf ein Verfahren zum spezifischen Nachweis eines Gerinnungsfaktors V mit einer erhöhten Stabilität gegenüber aktiviertem Protein C im aktivierten Zustand.

Das Gerinnungssystem im Blut sorgt zum einen für die Aufrechterhaltung des Blutstroms zu dem zu versorgenden Gewebe, zum anderen reagiert es auf Verletzungen mit einem Wundverschluß und sorgt damit für die Erhaltung der Integrität des Organismus. Bei der Aktivierung der Gerinnung entsteht über ein kaskaden-ähnliches System sich schrittweise aktivierender Proteasen letztlich die aktive Protease Thrombin. Die anfänglich nur sehr langsame Thrombinbildung wird durch Thrombin selbst beschleunigt, in dem es die Kofaktoren Faktor V und Faktor VIII durch proteolytische Spaltung aktiviert. Diese aktivierten Kofaktoren bilden mit den Proteasen Faktor Xa bzw. IXa aktive Enzym/Kofaktor-Komplexe auf Phospholipidoberflächen, deren Aktivität um den Faktor ca. 1000 höher ist als der singulären Proteasen. Durch diese positive Rückkopplung kommt es quasi explosionsartig zur Entstehung großer Mengen an Thrombin. Thrombin setzt Fibrinogen zu Fibrin um, das im Normalfall zum Wundverschluß und zur Wundheilung führt. Um eine lebensbedrohende Ausweitung der Gerinnung zu verhindern, die zu einem Verschluß des Gefäßsystems im Körper, also zu Thrombosen führen würden, müssen sowohl die aktive Protease als auch die Nachlieferung der Protease unterbunden werden. Aktive Proteasen werden im Körper durch Protease-Inhibitoren durch die Ausbildung kovalenter Komplexe neutralisiert. Die Unterbrechung des Nachschubes wird durch Thrombin selbst initiiert. Thrombin bindet hierzu an das Membranprotein Thrombomodulin und setzt das Proenzym Protein C zur aktiven Protease Protein Ca (APC) um. APC seinerseits bildet mit dem Kofaktor Protein S einen Komplex, der die aktiven Kofaktoren Faktor Vllla und Faktor Va proteolytisch spaltet und dadurch inaktiviert. APC unterbricht somit die starke Stimulierung durch diese Kofaktoren.

Dieses oben beschriebene Protein C/Protein S-System stellt einen wichtigen antikoagulatorischen Mechanismus dar. Dies wird dadurch bestätigt, daß Personen mit erblichen oder erworbenen Mängeln oder Defekten an Protein C oder Protein S mit hoher Wahrscheinlichkeit Thrombosen, insbesondere rezidivierende venöse Thrombosen, erleiden (Esmon, C.T. TCM 2: 214-219, 1992).

Neben Protein C und Protein S können weitere Faktoren die Aktivität des Systems beeinflussen, so von Willebrand Faktor und Faktor IXa (Rick, M.E. et al. J. Lab. Clin. Med. 115: 415-421, 1990), die Faktor Vllla vor proteolytischem Abbau schützen können. Erworbene Störungen können auch auf die Entstehung von Lupus anticoagulants zurückgehen. Dies sind gegen Phospholipide gerichtete Antikörper, die die zur Funktion notwendige Anbindung der Protease/Kofaktor Komplexe an Phospholipid-Oberflächen stören (Amer, L. et al. Thromb. Res. 57: 247-258, 1990).

In jüngster Zeit wurde eine Variante des Faktors V beschrieben, die im aktivierten Zustand (Faktor Va) nicht mehr oder zumindest nur sehr schlecht durch APC inaktiviert werden kann (Bertina, R.M. et al. Nature 369: 64-67, 1994). Dieser Defekt wird auch als "F.V-Leiden" bezeichnet und beruht auf einem Austausch von Arg 506 durch GIn in der Spaltregion für APC. Die Bedeutung dieser Mutation als Ursache für ein erhöhtes Thromboserisiko wurde in mehreren Studien in jüngster Zeit belegt.

Bisher stehen zwei Methoden zum Nachweis dieses veränderten Faktor V zur Verfügung. Zum ersten ist dies die Genomanalyse mittels Polymerase-Ketten-Reaktion ("polymerase chain reaction", PCR). Diese Methodik ist bekanntermaßen sehr aufwendig, nur in Speziallabors durchführbar und relativ teuer. Zudem erfaßt sie nur die bisher bekannte Mutation. Es ist jedoch vorstellbar, daß Mutationen auch an anderen Stellen Faktor Va gegen die Spaltung durch APC stabilisieren. Daher ist zusätzlich zu der sehr speziellen PCR-Methodik ein funktioneller Test unbedingt notwendig.

Ein solcher funktioneller Test, der auf einer bekannten Modifikation (Amer et al., 1990) eines in der Gerinnung üblichen Screening Verfahrens, der aktivierten partiellen Thromboplastin Zeit (APTT) beruht, wurde schon beschrieben. Zur Bestimmung der APTT wird dabei eine Plasmaprobe mit einem gleichen Volumenanteil eines Reagenzes in Kontakt gebracht, das einen Oberflächenaktivator, etwa Silica, Kaolin oder Glas und Phospholipide enthält. Dieses Gemisch wird wenige Minuten bei +37 °C inkubiert. Während dieser Zeit werden die nicht Calcium-abhängigen Faktoren des Gerinnungssystems (Faktor XII, Faktor Xl und Faktor IX) aktiviert. Nach Zugabe von Calciumionen wird die restliche Gerinnungskaskade aktiviert und es bildet sich Thrombin. Die entstandene Thrombinmenge wird dann entweder durch die Umsetzung des natürlichen Substrats Fibrinogen zu einem Gerinnsel oder durch die Freisetzung eines Chromophors aus einem chromogenen Substrat bestimmt. In Modifikation dieser APTT nach Amer, L. et al. (1990) wird gleichzeitig mit den Calciumionen aktiviertes Protein C zugegeben. Da, wie oben beschrieben, APC die Cofaktoren Vllla und Va zerstört, kommt es zu einer Verzögerung der Thrombinbildung, die abhängig ist von der Funktionalität des Protein C/Protein S Systems. Diese Modifikation wird im folgenden mit APC-Zeit (APCT) bezeichnet.

Die APCT in ihrer bisher genutzten Form ist nicht geeignet die erhöhte Stabilität des Faktor V gegenüber APC spezifisch nachzuweisen (siehe Beispiel 1). In das Meßergebnis gehen außer dem Protein C, das ja exogen in aktivierter Form zugegeben wird, alle oben beschriebenen Faktoren aus der Probe ein, die die Funktionalität des Protein C/Protein S-Systems beeinflussen. So kann eine Mutation im Faktor V, speziell wenn der Patient diesbezüglich heterozygot ist, nicht von einem Protein-S-Defekt oder -Mangel unterschieden werden. Ebenso können Autoantikörper gegen Protein S oder Protein C diesen Effekt vortäuschen.

Der Erfindung lag somit die Aufgabe zugrunde, Verfahren zu finden, mit dem Faktor V, der eine erhöhte Stabilität gegenüber dem Abbau durch APC aufweist, nachgewiesen werden kann.

Die Lösung der Aufgabe erfolgt durch die Bereitstellung der in den Patentansprüchen beschriebenen Ausführungsformen.

Es wurde gefunden, daß bei Verdünnung von Plasmen mit einem Faktor V armen Reagenz, das Protein S und die Gerinnungsfaktoren X und Prothrombin enthält, der Einfluß von Protein S eliminiert werden kann, während die veränderte Stabilität des Faktor V gegenüber APC weiterhin entscheidend für das Meßergebnis ist. Darüber hinaus können in diesem Verfahren heparinisierte Proben und auch Proben von Patienten unter Marcumar-Therapie verwendet werden, was in dem bisherigen funktionellen Tests nicht möglich ist.

Im einfachsten Fall handelt es sich bei dem Reagenz mit dem die Probe verdünnt wird um humanes Faktor V-Mangelplasma. Nach Aktivierung des Faktor V der Probe wird dieser durch aktiviertes Protein C oder eine sich gleichsinnig verhaltende Protease zerstört und die residuale Faktor Va-Aktivität bestimmt.

Die Aktivierung des Faktor V der Probe, dessen Zerstörung und die Nachweisreaktion beruhen im einfachsten Fall auf der Auslösung der Gerinnung in der Probe unter Zusatz von aktiviertem Protein C (APC). Der Anteil des Faktor V-Mangelplasmas (F.V-MP) am Probenvolumen muß für das jeweilige Testverfahren optimiert werden (siehe Beispiele) und liegt bei 50 - 95 %, vorzugsweise jedoch im Bereich von 60-80 %. Für den gesamten Testansatz bedeutet dies, daß der Anteil der Plasmaprobe höchstens 20 %, vorzugsweise jedoch kleiner oder gleich 10 % beträgt.

Das erfindungsgemäße Verfahren kann somit auf einer Modifikation der APTT beruhen, in der zunächst die Gerinnungsfaktoren XII, Xl und IX aktiviert werden. Durch Zugabe eines Gemisches von APC und Calciumchlorid wird die Bildung von Thrombin ausgelöst, das Faktor V aktiviert. Durch die gleichzeitige Anwesenheit von APC wird Faktor Va inaktiviert, wodurch die Geschwindigkeit der Gerinnselbildung verlangsamt wird. Mit Erhöhung des Anteils an Faktor V-Mangelplasma am Probenvolumen wird durch den Faktor V-Mangelplasma einerseits die Gerinnungszeit verlängert, andererseits aber der Einfluß eines Protein S-Mangels verringert. So wird in Beispiel 2 gezeigt, daß ab ca. 70 % Anteil Faktor V-Mangelplasma am Probenvolumen die Gerinnungszeiten eines normalen Plasmas und eines Protein S-Mangelplasmas gleich sind, während in einem homozygoten oder heterozygoten Plasma, das einen veränderter Faktor V mit einer erhöhten Stabilität gegenüber APC aufweist, die Differenzen der Gerinnungszeiten relativ zum normalen Plasma eher noch zunimmt. Damit verbessert sich die Differenzierbarkeit beider Effekte gegenüber dem bisherigen Stand der Technik erheblich.

Anstelle der APTT kann auch die Thromboplastin-Zeit (PT) angewandt werden (Beispiel 3). Reagenzien für die PT enthalten ein Gemisch aus Thromboplastin, einem membrangebundenen Cofaktor, Phospholipide und Calciumchlorid. Nach Zugabe zur Probe wird Faktor VII zunächst autokatalytisch gespalten zu Faktor Vlla. Faktor Vlla zusammen mit Thromboplastin als Cofaktor sorgt dann für die Aktivierung von Faktor X und dieser für die Bildung von Thrombin, das wieder Faktor V aktiviert. Wird gleichzeitig mit dem Thromboplastin-Reagenz aktiviertes Protein C zugegeben, so wird auch hier die Gerinnungszeit verlängert. Ist jedoch Faktor Va schwerer proteolytisch angreifbar, ist diese Verlängerung weniger ausgeprägt. Da die Gerinnungszeiten durch diesen kurzen Aktivierungsweg sehr kurz sind, wurde in Beispiel 3 der Gehalt an Thromboplastin ausverdünnt. Die daraus resultierende Verlängerung der Gerinnungszeit führt zu einer noch deutlicheren Verlängerung der Gerinnungszeit in Anwesenheit von APC. Dadurch wird die Differenzierbarkeit (Signal/Hintergrund-Verhältnis) zwischen normalem und veränderten Faktor V verbessert. Beispiel 3 zeigt auch, daß durch eine erhöhte Verdünnung der Probe mit Faktor V-Mangelplasma der Einfluß eines Protein S-Mangels neutralisiert wird. Im Gegensatz zur modifizierten APTT wird jedoch eine höhere Verdünnung der Probe benötigt.

Das erfindungsgemäße Verfahren kann auch in einer Modifikation der RVVT ("Russell Viper Venom Time") angewandt werden. Die RVVT beruht auf der Verwendung von Enzymen aus dem Schlangengift von Vipera russellii. Dieses enthält Proteasen, die die humanen Gerinnungsfaktoren X und V durch proteolytische Spaltung aktivieren. Dieses an sich bekannte Verfahren umgeht somit den extrinsischen und intrinsischen Weg der Gerinnung und wird zur Erfassung von Faktor X-, Faktor V- und Prothrombin-Mangel, aber auch in der Lupus-Diagnostik verwendet (Thiagarajan, P. et al., 1986). Neuerdings wird das Verfahren in Verbindung mit APC gleichsinnig wie der auf der APTT-basierende Test zur Bestimmung von Störungen des Protein C/Protein S-Systems eingesetzt. Aber auch für dieses Verfahren gilt, daß neben dem Faktor V-Defekt sich auch andere Störungen, etwa ein Protein S-Mangel, gleichsinnig verhalten und daher nicht in diesem Test voneinander unterschieden werden können. Beispiel 4 zeigt, daß die Anwendung des erfindungsgemäßen Verfahren demgegenüber eine spezifische Differenzierung erlaubt, wobei vorzugsweise der Anteil des F.V-MP am Probenvolumen 75 % beträgt.

Zusätzlich zur spezifischeren Differenzierung zeigt Beispiel 5, daß durch Zusatz von Heparin-neutralisierenden Substanzen zum F.V-MP Heparin in der Probe neutralisiert werden kann. Dies ist ein weiterer wesentlicher Vorteil der Methode gegenüber dem Standardverfahren (APCT). Denn gerade bei Patienten mit einer Thrombose besteht ein großes Interesse daran, die Ursache des Vorfalls zu ergründen. Diese Patienten stehen aber meist unter Antikoagulantien, wie eben Heparin.

Nach Heparin werden viele Patienten nach einer Thrombose auf Marcumar-Therapie gesetzt. Marcumar ist ein Vitamin K-Antagonist und führt zur unvollständigen Synthese von Gerinnungsfaktoren. Die Untersuchungen in Beispiel 6 legen die Vermutung nahe, daß mit dem erfindungsgemäßen Verfahren auch in Patienten unter Marcumar-Therapie nunmehr ein F.V-Defekt nachzuweisen ist.

In Alternative zur Zugabe von aktiviertem Protein C, kann auch Protein C im nicht-aktivierten Zustand zugegeben werden oder das Protein C des Faktor V-Mangelplasmas genutzt und aktiviert werden. Die geschieht beispielsweise durch Zugabe von Protein C-Aktivatoren aus Schlangengiften der Gattung Agkistrodon, wie auch in deutschen Patentanmeldung P 44 27 785.7 aufgeführt (siehe Beispiel 7).

Bei Anwendung von traditionellen Gerinnungsverfahren, wie der APTT, der PT und der RVVT, erfolgt die Bestimmung der Gerinnungsaktivität durch mechanische, mechano-optische oder optische Detektion der Gerinnselbildung. Das erfindungsgemäße Verfahren kann auch mit moderneren chromogenen Verfahren, beispielsweise durch Bestimmung des Umsatzes eines chromogenen Thrombinsubstrates kombiniert werden.

Anstelle von F.V-Mangelplasma kann ein Reagenziensystem aus gereinigten Gerinnungsfaktoren verwendet werden. Das Prinzip der Methode beruht in Analogie zur ersten Variante darauf, daß Faktor Va der Geschwindigkeitslimitierende Faktor der Prothrombin-Aktivierung ist. Durch die gleichzeitige Anwesenheit von APC wird der aktivierte Faktor V zerstört und dadurch die Gerinnungszeit verlängert. Die Probe wird somit mit einem Faktor V-abhängigen Prothrombin Aktivator sowie einem Faktor V-Aktivator, beispielsweise aus dem Schlangengift von Vipera russellii, in Kontakt gebracht und die Aktivierung von zugegebenem Prothrombin bestimmt, wozu in der Gerinnungsdiagnostik bekannte Verfahren, wie etwa die Umsetzung von Fibrinogen oder bei chromogenen Verfahren die Umsetzung eines chromogenen Thrombinsubstrates zur Messung verwendet werden.

Es ist sinnvoll, dem Testansatz für die Gerinnungsenzyme wichtige Cofaktoren wie Calciumchlorid und Phospholipide zuzusetzen. Zur Ausblendung von Lupus anticoagulant aus dem Plasma ist es weiterhin sinnvoll, Phospholipide in einer hohen Konzentration, bevorzugterweise zwischen 0,3 und 0,001 %, besonders bevorzugterweise zwischen 0,001 und 0,01 %, zuzusetzen. Zur Entfaltung der Protein Ca-Aktivität sollte weiterhin Phosphatidylethanolamin anteilig in den Phospholipiden enthalten sein (Horie, S. et al., 1994). Zur Vermeidung von Störungen der Meßergebnisse aufgrund von Protein S-Mangel und/oder -Defekt in der Probe wird weiterhin sinnvollerweise ein Reagenzsystem Protein S im Konzentrationsbereich von 0,1 - 5 E/ml (bezogen auf den Testansatz), besonders bevorzugt im Bereich von 1 - 2 E/ml, enthalten. Schließlich muß aktiviertes Protein C zugesetzt werden, über dessen Konzentration das Meßsignal letztlich gesteuert wird. Üblicherweise wird die APC-Konzentration im Testansatz im Bereich von 0,01 - 1 E/ml liegen.

Dieses Reagenziensystem umfaßt somit mindestens die Komponenten Faktor X, Protein S, Prothrombin, die zur Gerinnung notwendigen Phospholipide, Calciumchlorid , aktiviertes Protein C bzw. alternativ nicht-aktiviertes Protein C und getrennt davon einen Protein C-Aktivator. Das Reagenziensystem kann entweder kombiniert als Monoreagenz oder zur Erhöhung der Stabilität in getrennter Form zur Anwendung kommen, wie beispielsweise als Reagenz 1, das Faktor X, Protein S, Prothrombin, Phospholipide beinhaltet und als Reagenz 2, das aktiviertes Protein C und Calciumchlorid beinhaltet. Die Probe wird mit dem Reagenziensystem gemischt. Die Gerinnung wird ausgelöst entweder über die Aktivierung der Kontaktphase nach Art der APTT, durch direkte Aktivierung von Faktor X und/oder Faktor V mittels RVV oder durch spurenweise Aktivierung von Thrombin durch Zugabe von Prothrombin spaltenden Enzymen wie beispielsweise aktivem Faktor X oder Ecarin aus dem Schlangengift von Echis carinatus. Bei der Aktivierung mittels RVV bzw. Ecarin werden diese Enzyme vorzugsweise zusammen mit APC und Calciumchlorid in Reagenz 2 als Startreagenz verwendet. Die Detektion erfolgt dann vorzugsweise durch Bestimmung der Thrombinbildung durch Umsatz eines Thrombin-spezifischen, chromogenen Substrats.

Weiterhin kann anstelle von aktiviertem Protein C nicht-aktiviertes Protein C verwendet werden, welches erst im Testansatz durch geeignete Aktivatoren, beispielsweise aus dem Schlangengift der Gattung Agkistrodon, erhältlich unter dem Handelsnamen Protac®, aktiviert wird. Ein solches Reagenzsystem umfaßt dann beispielsweise Reagenz 1 ( beinhaltend Protein S, Faktor X, Protein C, Prothrombin und Phospholipide) sowie Reagenz 2 (beinhaltend Protac®, RW, Calciumchlorid und ein Thrombin-Substrat). Das Reagenz 1 kann auch nur ein Faktor V-Mangelplasma sein, wobei dann aber die Phospholipide dem Reagenz 2 zugeteilt werden. Um den Effekt des endogenen Protein C zu verstärken, kann das Reagenz 2 auch geteilt und nach einer Vorinkubation des Gemisches aus Probe und Reagenz 1 mit einem Reagenz 2a aus Protac® und Phospholipiden zur Aktivierung des Protein C die Gerinnungsreaktion und Nachweisreaktion der Faktor V-Stabilität durch Zusatz von Reagenz 2b, beinhaltend RW und Calciumchlorid ausgelöst werden. Die Zugabe der Phospholipide ist dabei willkürlich und die weitere Zugabe eines chromogenen Substrats nur abhängig von der gewünschten Auswertetechnik.

Weiterhin kann in einem Reagenziensystem aus gereinigten Faktoren, Faktor X durch nicht-humane Faktor Va-abhängige Prothrombin-Aktivatoren, etwa aus Schlangengiften (zur Übersicht siehe: Rosing, J. and Tans, G., 1991), ersetzt werden. Dies ist ebenfalls in Kombination mit aktiviertem Protein C oder mit im Testansatz aktiviertem Protein C dazu geeignet, spezifisch einen Faktor V in Plasmaproben nachzuweisen, der eine erhöhte Stabilität gegenüber dem proteolytischen Abbau durch aktiviertes Protein C besitzt. So könnte ein Reagenziensystem aus folgenden Komponenten bestehen: Reagenz 1 aus Protein S, Protein C, Prothrombin und Phospholipiden sowie Reagenz 2 aus Protac®, RW oder RW-V (die nur Faktor V aktivierenden Protease aus RW), Faktor V-abhängigem Prothrombin-Aktivator, Calciumchlorid und einem Thrombin-Substrat, bzw. das Thrombin-Substrat als Reagenz 3.

Reagenziensysteme, die auf einer APTT basieren, sind von der Konzentration an Faktor VIII in der Probe abhängig, wie in Beispiel 8 aufgeführt ist. Es wurde überraschenderweise gefunden, daß ein Faktor V-Mangelplasma, das Faktor VIII in physiologischen Konzentrationen (0,7 - 1,4 Einheiten/ml) enthält, gegenüber einem Mangelplasma, das keinen Faktor VIII enthält, weniger von der Konzentration an Faktor VIII der Probe abhängig ist. Damit wird die Differenzierung zwischen normalen Plasmen mit hohem Faktor VIII-Gehalt und Plasmen mit normalem Faktor VIII-Gehalt aber mit Faktor V-Leiden verbessert. In Reagenziensystemen, die auf einer APTT basieren werden daher bevorzugterweise Faktor V-Mangelplasma und/oder Reagenzien verwendet, die Faktor VIII in Konzentrationen zwischen 0 und 4 E/ml, besonders bevorzugterweise im Konzentrationsbereich zwischen 0,7 und 1,3 E/ml enthalten, bzw. durch Zusatz substituiert werden.

### Verwendete Abkürzungen:

| | |
|---|---|
| APCT | aktivierte Protein C Zeit |
| APCV | aktivierte Protein C Zeit unter Mischung der Probe mit Faktor V-Mangelplasma |
| APTT | aktivierte, partielle Thromboplastin Zeit |
| Arg | Arginin |
| F.V-MP | humanes Faktor V-Mangelplasma |
| F.V-Leiden | Aminosäureaustausch Arg -> GIn an Position 506 im Faktor V |
| Gln | Glutamin |
| PC-MP | humanes Protein C-Mangelplasma |
| PS-MP | humanes Protein S-Mangelplasma |
| RVVT | Russell Viper Venom Time |
| SHP | Standard-Human-Plasma (ein Pool von normalem, humanem Plasma) |
| Tris | Tris-(hydroxymethyl)-aminomethan |

### Beispiel 1

### Limitierung des Standes der Technik zum funktionellen Nachweis eines F.V-Leidens oder gleichsinnigen Defekts

Die Bestimmung der Gerinnungszeit erfolgte an einem automatischen Koagulometer (Behring Fibrintimer A, Fa. Behringwerke; Fa. Behringwerke AG, Marburg). Alle Reagenzien waren von der Fa. Behringwerke AG. Als Plasmapool normaler Blutspender wurde SHP verwendet.

Die Bestimmung der APTT erfolgt nach Vorschrift: 1 Abfüllung Pathromtin® für 5 ml, ein Phospholipidgemisch aus humaner Plazenta, wurde in 5 ml Kaolinsuspension als Oberflächenaktivator gelöst. Die Calciumchloridlösung (25 mM) wurde vor Gebrauch auf +37 °C erwärmt.

In ein Meßröhrchen wurden nacheinander pipettiert
100 µl Pathromtin®
100 µl Plasmaprobe.

Anschließend wurde bei +37 °C für 2 Minuten inkubiert und durch Zugabe von 100 µl Calciumchlorid-Lösung die Gerinnungszeit gestartet. Die Gerinnungszeit wurde bei 405 nm bestimmt.

Die Bestimmung der APCT erfolgte mit den APC-Sensitivitätsreagenzien der Fa. Behringwerke. Wie bei der APTT wurde das Aktivatorreagenz hergestellt. Das Startreagenz aus Calciumchlorid und aktiviertem Protein C wurde in 5 ml destilliertem Wasser gelöst und vor Gebrauch auf +37 °C erwärmt.

In ein Meßröhrchen wurden nacheinander pipettiert
100 µl Pathromtin®
100 µl Plasmaprobe.

Anschließend wurde bei +37 °C für 2 Minuten inkubiert und durch Zugabe von 100 µl Startreagenz die Gerinnungszeit gestartet. Die Gerinnungszeit wurde bei 405 nm bestimmt.

Die APTT und APCT wurde in folgenden humanen Citrat-Plasmen bestimmt: in einem Pool von normalen Blutspendern (SHP), in Plasmen mit einem Mangel an Protein C (PC-MP) bzw. Protein S (PS-MP), sowie in einem Plasma mit einem homozygoten F.V-Leiden-Defekt. Zur Simulation eines heterozygoten Defekts, bei dem ca. 50 % des plasmatischen Faktor V intakt bzw. in der F.V-Leiden-Form vorliegen, wurde das homozygote F.V-Leiden Plasma 1:1 mit SHP gemischt.

In Tabelle 1 sind die erhaltenen Gerinnungszeiten sowie die Differenzen der APCT relativ zu SHP dargestellt. Für die APTT lagen die Werte alle innerhalb des Normbereichs (≤ 40 sec), eine Voraussetzung zur Durchführung der APCT. In der APCT sind solche Gerinnungszeiten pathologisch, die gegenüber dem normalen Plasma verkürzt sind. Dies trifft für Protein C Mangel nicht zu. Da in der APCT das aktivierte Protein C exogen zugefügt wird, kann das Protein C der Probe den Test nicht beeinflussen. Hingegen führt Protein S-Mangel zu einer Verkürzung der Gerinnungszeit, die in diesem Fall sogar etwas stärker ausgeprägt ist als bei einem heterozygoten F.V-Leiden Defekt. Bei einem homozygoten F.V-Leiden Defekt ist die Verkürzung in der APCT am stärksten ausgeprägt. Die Unterschiede zwischen Protein S-Mangel und einem homozygoten F.V-Leiden Defekt sind nur schwach ausgeprägt. Diese Unterschiede werden in der Praxis noch weiter verwischt, indem abhängig von der Zeitdauer der Blutentnahme bis zur Bestimmung kontinuierlich die Faktoren V und VIII denaturieren. Dies führt zu einer Verlängerung der Gerinnungszeit, die der beobachteten Verkürzung der Gerinnungszeit entgegenwirkt.

Das bisher zur Verfügung stehende Verfahren, d.h. die APCT ist somit nicht geeignet, einen F.V-Leiden-Defekt oder einen ähnlichen Defekt im Faktor V spezifisch nachzuweisen, der zu einer erhöhten Stabilität gegenüber aktiviertem Protein C führt.

**Tabelle 1: APTT und APCT verschiedener Plasmen mit Mangel- bzw. Defekten die die Funktionalität des Protein C/Protein S-Systems beeinflussen. Angabe der Gerinnungszeiten (absolut) bzw. die Differenz der APCT zu SHP in Sekunden. SHP = normales Plasma; PC-MP = Protein C-Mangelplasma, PS-MP = Protein S-Mangelplasma, F.V-L1/1 = Plasma mit homozygoten F.V-Leiden Defekt, F.V-L 1/2 = Plasma mit heterzygoten F.V-Leiden Defekt.**

| | absolut | | Differenz |
|---|---|---|---|
| Plasma | APTT | APCT | APCT |
| SHP | 34,0 | 120,6 | - |
| PC-MP | 38,9 | 216,1 | 95,5 |
| PS-MP | 37,6 | 78,9 | -41,7 |
| F.V-L 1/2 | 34,8 | 83,7 | -36,9 |
| F.V-L 1/1 | 32,5 | 61,8 | -58,8 |

### Beispiel 2

### Optimierung des Verfahrens auf Basis einer modifizierten APTT

Die Bestimmung der Gerinnungszeit erfolgte an einem automatischen Koagulometer (Behring Fibrintimer A, Fa. Behringwerke; Fa. Behringwerke AG, Marburg). Alle Reagenzien waren von der Fa. Behringwerke AG. Als Plasmapool normaler Blutspender wurde SHP verwendet.

Für die Bestimmung der Gerinnungszeit wurde 1 Abfüllung Pathromtin® für 5 ml in 5 ml Kaolinsuspension gelöst. Das Startreagenz aus den APC-Sensitivitätsreagenzien (beinhaltend Calciumchlorid und aktiviertes Protein C) wurde in 5 ml destilliertem Wasser gelöst und vor Gebrauch auf +37 °C erwärmt. Faktor V-Mangelplasma wurde in 1 ml destilliertem Wasser gelöst.

In ein Meßröhrchen wurden nacheinander pipettiert
x µl Plasmaprobe
y µl F.V-MP
100 µl Pathromtin®.

Anschließend wurde bei +37 °C für 3 Minuten inkubiert und durch Zugabe von 100 µl Startreagenz die Gerinnungszeit gestartet. Die Gerinnungszeit wurde bei 405 nm bestimmt.

Die Volumina x und y wurden so gewählt, daß das Gesamtvolumen (x+y) exakt 100 µl betrug. Die Gerinnungszeiten wurden in folgenden humanen Citrat-Plasmen bestimmt: in einem Pool von normalen Blutspendern (SHP), in Plasmen mit einem Mangel an Protein C (PC-MP) bzw. Protein S (PS-MP), sowie in einem Plasma mit einem homozygoten F.V-Leiden-Defekt. Zur Simulation eines heterozygoten Defekts, bei dem ca. 50 % des plasmatischen Faktor V intakt bzw. in der F.V-Leiden-Form vorliegen, wurde das homozygote F.V-Leiden Plasma 1:1 mit SHP gemischt.

In Tabelle 2 sind die erhaltenen Gerinnungszeiten sowie die Differenzen der Gerinnungszeiten relativ zu SHP aufgeführt. Mit steigendem Anteil von F.V-MP am Probenvolumen werden die Gerinnungszeiten aufgrund des zunehmenden Faktor V-Mangels länger. Während aber die Differenz zwischen SHP und PC-MP nahezu unverändert bleibt, wird der Abstand zwischen SHP und PS-MP immer kürzer und ab ca. 70 %-igem Anteil von F.V-MP im Testansatz ist kein Unterschied mehr zu vermerken. Der Protein S-Mangel in der Probe wird somit durch das Protein S im F.V-MP ausreichend neutralisiert. Die Plasmen mit homozygotem bzw. (simulierten) heterozygotem F.V-Leiden Defekt verhalten sich jedoch genau entgegengesetzt. Hier nimmt die Differenz der Gerinnungszeiten relativ zu SHP zu.

Das Verfahren erlaubt somit nicht nur PS-Mangel auszublenden, es verstärkt den durch F.V-Leiden verursachten Effekt sogar. Damit werden auch Störungen durch längere Standzeiten der Plasmen nicht so leicht wie in bisherigen Verfahren (Beispiel 1) zu einem Verwischen der Unterschiede zwischen PS-Mangel und F.V-Defekt führen. Weiterhin ist zu erwarten, daß in diesem Verfahren Autoantikörper gegen PS neutralisiert werden, da ja der PS-Effekt eliminiert wird. Aber auch Autoantikörper gegen PC oder PC/PS-Phospolipid-Komplexe werden bei hoher Verdünnung der Probe mit dem F.V-MP allein aufgrund der Verdünnung kaum noch zu bemerken sein.

**Tabelle 2: Gerinnungszeiten verschiedener Plasmen mit Mangel- bzw. Defekten die die Funktionalität des Protein C/Protein S-Systems beeinflussen im Verfahren auf Basis einer modifizierten APTT in Abhängigkeit von dem Anteil an F.V-MP im Probenvolumen. Angabe der erhaltenen Gerinnungszeiten (A) bzw. deren Differenzen (B) zu SHP in Sekunden. SHP = normales Plasma; PC-MP = Protein C-Mangelplasma, PS-MP = Protein S-Mangelplasma, F.V-L1/1 = Plasma mit homozygoten F.V-Leiden Defekt, F.V-L1/2 = Plasma mit heterzygoten F.V-Leiden Defekt.**

| | | Anteil von F.V-Mangelplasma | | | |
|---|---|---|---|---|---|
| | Plasma | 50% | 60% | 70% | 75% |
| (A) | SHP | 130,9 | 149,7 | 171,1 | 184,3 |
| | PC-MP | 181,3 | 194,8 | 183,7 | 231,5 |
| | PS-MP | 109,8 | 140,0 | 175,5 | 198,3 |
| | F.V-L1/1 | 69,1 | 78,1 | 89,0 | 98,2 |
| | F.V-L1/2 | 90,3 | 100,9 | 114.8 | 124,9 |
| (B) | PC-MP | 50,4 | 45,1 | 12,6 | 47,2 |
| | PS-MP | -21,1 | -9,7 | 4,4 | 14,0 |
| | F.V-L1/1 | -61,8 | -71,6 | -82,1 | -86,1 |
| | F.V-L1/2 | -40,6 | -48,8 | -56,3 | -59,4 |

### Beispiel 3

### Optimierung des Verfahrens auf Basis einer modifizierten Thromboplastin-Zeit

Die Bestimmung der Gerinnungszeit erfolgte an einem automatischen Koagulometer (Behring Fibrintimer A, Fa. Behringwerke; Fa. Behringwerke AG, Marburg). Alle Reagenzien waren von der Fa. Behringwerke AG. Als Plasmapool normaler Blutspender wurde SHP verwendet.

Für die Bestimmung der Gerinnungszeit wurde Thromboplastin®, ein PT-Reagenz aus humaner Plazenta, nach Vorschrift des Herstellers in destilliertem Wasser gelöst und 1:2000 mit einem Puffer, bestehend aus 50 mM Tris/HCI, 0,01 % Phospholipon-25 (ein Phospholipid-Gemisch aus Sojabohnen), 10 mM Calciumchlorid, 0,4 E/ml APC, pH 7,4 verdünnt. Das Reagenz wurde vor Gebrauch auf +37 °C erwärmt. Faktor V-Mangelplasma wurde in 1 ml destilliertem Wasser gelöst.

In ein Meßröhrchen wurden nacheinander pipettiert
x µl Plasmaprobe
y µl F.V-MP
100 µl Thromboplastin ®1:2000.

Anschließend wurde bei +37 °C für 3 Minuten inkubiert und durch Zugabe von 100 µl Startreagenz die Gerinnungszeit gestartet. Die Gerinnungszeit wurde bei 405 nm bestimmt.

Die Volumina x und y wurden so gewählt, daß das Gesamtvolumen (x+y) exakt 100 µl betrug. Dabei entspricht die Variante mit 0 pl F.V-MP einer Variante der APCT auf Basis der Thromboplastin-Zeit. Die Gerinnungszeiten wurden in den gleichen Proben wie in Beispiel 2 bestimmt.

In Tabelle 3 sind die erhaltenen Gerinnungszeiten sowie die Differenzen der Gerinnungszeiten relativ zu SHP aufgeführt.

Mit steigendem Anteil von F.V-MP am Probenvolumen werden die Gerinnungszeiten aufgrund des zunehmenden Faktor V-Mangels länger. Allerdings konnte selbst bei einem bis zu 75 %-igen Anteil an F.V-MP im Probenvolumen der Protein S-Einfluß nicht vollständig neutralisiert werden, so daß noch höhere Konzentrationen verwendet werden müssen. Weiterhin kann über die Wahl der Puffersubstanzen, der lonenstärken und der Phospholipid-Konzentration die Reaktivität noch weiter optimiert werden. Trotzdem ist zu beobachten, daß sich die Differenzen der Gerinnungszeiten zwischen PS-MP und F.V-Leiden, sowohl homo- als auch heterozygot vergrößern, die Differenzierbarkeit zwischen beiden Störungen des Protein C/PS-Systems mit dem Verfahren auch auf Basis der PT somit verbessert wird.

**Tabelle 3: Gerinnungszeiten verschiedener Plasmen mit Mangel- bzw. Defekten die die Funktionalität des Protein C/Protein S-Systems beeinflussen im Verfahren auf Basis einer modifizierten PT in Abhängigkeit von dem Anteil an F.V-MP im Probenvolumen. Angabe der erhaltenen Gerinnungszeiten (A) bzw. deren Differenzen (B) zu SHP in Sekunden. SHP = normales Plasma; PC-MP = Protein C-Mangelplasma, PS-MP = Protein S-Mangelplasma, F.V-L1/1 = Plasma mit homozygoten F.V-Leiden Defekt, F.V-L1/2 = Plasma mit heterzygoten F.V-Leiden Defekt**

| | | Anteil von F.V-Mangelplasma | | | | |
|---|---|---|---|---|---|---|
| | Plasma | 50% | 60% | 70% | 75% | 80% |
| (A) | SHP | 128,1 | 138,9 | 148,3 | 160,0 | 171,4 |
| | PC-MP | 151,7 | 189,7 | 179,2 | 202,5 | 224,1 |
| | PS-MP | 75,7 | 83,3 | 104,3 | 121,4 | 144,3 |
| | F.V-L1/1 | 67,7 | 70,0 | 83,3 | 89,2 | 104,4 |
| | F.V-L 1/2 | 74,8 | 78,6 | 87,7 | 98,8 | 120,2 |
| (B) | PC-MP | 23,6 | 50,8 | 30,9 | 42,5 | 52,7 |
| | PS-MP | -52,4 | -55,6 | -44,0 | -38,6 | -27,1 |
| | F.V-L1/1 | -60,4 | -68,9 | -65,0 | -70,8 | -67,0 |
| | F.V-L1/2 | -53,3 | -60,3 | -60,6 | -61,2 | -51,2 |

### Beispiel 4

### Erfindungsgemäßes Verfahren auf Basis einer modifizierten RW-Zeit

Die Bestimmung der Gerinnungszeit erfolgte an einem äutomatischen Koagulometer (Behring Fibrintimer A, Fa. Behringwerke; Fa. Behringwerke AG, Marburg). Als RW-Reagenz wurde das Reagenz LA-Confirm® der Fa. Gradipore, Australien, verwendet. Alle anderen Reagenzien waren von der Fa. Behringwerke AG.

Für die Bestimmung der Gerinnungszeit wurde 1 Abfüllung LA-Confirm® für 2 ml mit 2 ml Startreagenz aus den APC-Sensitivitätsreagenzien (beinhaltend Calciumchlorid und aktiviertes Protein C) gelöst und vor Gebrauch auf +37 °C erwärmt (= RVV/APC-Reagenz). Faktor V-Mangelplasma wurde in 1 ml destilliertem Wasser gelöst.

In ein Meßröhrchen wurden nacheinander pipettiert
x µl Plasmaprobe
y µl F.V-MP

und durch Zugabe von 100 µl RW/APC-Reagenz die Gerinnungszeit gestartet. Die Gerinnungszeit wurde bei 405 nm bestimmt.

Die Volumina x und y wurden so gewählt, daß das Gesamtvolumen (x+y) exakt 100 µI betrug. Dabei entspricht die Variante mit 0 µl F.V-MP einer Variante der APCT auf Basis des RVV-Assays. Die Gerinnungszeiten wurden in den gleichen Proben wie in Beispiel 2 bestimmt.

In Tabelle 4 sind die erhaltenen Gerinnungszeiten sowie die Differenzen der Gerinnungszeiten relativ zu SHP aufgeführt. Wie in den anderen Testvarianten (Beispiele 2 und 3) werden die Gerinnungszeiten mit steigendem Anteil von F.V-MP am Probenvolumen verlängert. Protein C-Mangelplasma ist immer gegenüber SHP verlängert. Die Differenz zwischen SHP und Protein S-Mangelplasma wird mit steigendem Anteil an F.V-MP geringer, der Effekt des F.V-Leiden hingegen größer. Ähnlich wie bei der Testvariante auf Basis der PT (Beispiel 3), wird der Protein S-Mangel mit den hier aufgeführten Anteilen an F.V-MP nicht vollständig neutralisiert, so daß der Anteil an F.V-MP noch größer 70 % gewählt werden muß. Aber die Differenzen zwischen einem Plasma mit F.V-Leiden, ob homo- oder heterozygot, mit Protein S-Mangel oder -Defekt, verstärken sich, so daß auch auf der Basis der RVVT das erfindungsgemäße Verfahren zu einer besseren Differenzierung eines gegenüber aktiviertem Protein C stabileren Faktors V von anderen Störungen des Protein C/Protein S-Systems führt.

**Tabelle 4: Gerinnungszeiten verschiedener Plasmen mit Mangel- bzw. Defekten, die die Funktionalität des Protein C/Protein S-Systems beeinflussen im erfindungsgemäßen Verfahren auf Basis einer modifizierten RVVT in Abhängigkeit von dem Anteil an F.V-MP im Probenvolumen. Angabe der erhaltenen Gerinnungszeiten (A) bzw. deren Differenzen (B) zu SHP in Sekunden. SHP = normales Plasma; PC-MP = Protein C-Mangelplasma, PS-MP = Protein S-Mangelplasma, F.V-L1/1 = Plasma mit homozygoten F.V-Leiden Defekt, F.V-L1/2 = Plasma mit heterzygoten F.V-Leiden Defekt.**

| | | Anteil von F.V-Mangelplasma | | | |
|---|---|---|---|---|---|
| Plasma | | 50% | 60% | 70% | 75% |
| (A) | SHP | 93,8 | 101,1 | 109,2 | 121,1 |
| | PC-MP | 122,4 | 127,4 | 139,2 | 149,4 |
| | PS-MP | 73,2 | 82,8 | 94,0 | 107,7 |
| | F.V-L1/1 | 48,7 | 53,2 | 60,0 | 67,2 |
| | F.V-L1/2 | 58,1 | 63,3 | 71,1 | 79,2 |
| (B) | PC-MP | 28,6 | 26,3 | 30,0 | 28,3 |
| | PS-MP | -20,6 | -18,3 | -15,2 | -13,4 |
| | F.V-L1/1 | -45,1 | -47,9 | -49,2 | -53,9 |
| | F.V-L1/2 | -35,7 | -37,8 | -38,1 | -41,9 |

### Beispiel 5

### Neutralisierung von Heparin durch Zusätze im Faktor V-Mangelplasma

Heparin wurde von Hoffmann-La Roche, Grenzach-Wyhlen, bezogen (Liquemin^{®} 25000), Polybren von der Fa. Ega-Chemie, Steinheim. Die übrigen Reagenzien und Geräte waren von der Fa. Behringwerke AG.

Standardhumanplasma bzw. ein Plasma mit einem Faktor V-Leiden Defekt wurde mit Heparin versetzt und die Gerinnungszeiten, wie unter Beispiel 2 aufgeführt, mit 75 % Anteil F.V-MP am Probenvolumen im erfindungsgemäßen Verfahren bestimmt. Zur Neutralisierung von Heparin in der Probe wurde das Faktor V-Mangelplasma zusätzlich mit 10 µg/ml Polybren versetzt.

In Tabelle 4 sind die erhaltenen Gerinnungszeiten in Anwesenheit von 0 bis 2 E/ml Heparin in den verschiedenen Testvarianten aufgeführt. Ohne Heparin-neutralisierenden Zusatz zum F.V-MP weichen die Gerinnungszeiten bereits in Anwesenheit oberhalb von 0,2 E/ml Heparin deutlich von der Probe ohne Heparin ab. Durch Zusatz von 10 µg/ml Polybren zum F.V-MP kann die Bestimmung des Faktor V-Defekts selbst in Anwesenheit bis zu 2 E/ml Heparin erfolgen. Damit ist im Gegensatz zum bisherigen Stand der Technik die Bestimmung des F.V-Leiden Defekts auch in heparinisierten Proben möglich.

**Tabelle 5: Neutralisierung von Heparin in einem normalen Plasma und in einem Plasma mit einem Faktor V-Leiden Defekt durch Zusatz von Polybren im erfindungsgemäßen Verfahren. Angabe der Gerinnungszeiten in Sekunden. SHP = normales Plasma; F.V-L = Plasma mit homozygoten F.V-Leiden Defekt.**

| | SHP | | F.V-L | |
|---|---|---|---|---|
| Heparin (IE/ml) | - PB | + PB | - PB | + PB |
| 0 | 185,0 | 195,7 | 96,8 | 100,9 |
| 0,2 | 206,4 | 190,5 | 104,1 | 101,8 |
| 0,4 | >300 | 195,3 | 143,2 | 100,0 |
| 0,6 | >300 | 193,8 | >300 | 100,3 |
| 0,8 | >300 | 198,9 | >300 | 99,6 |
| 1,0 | >300 | 198,9 | >300 | 100,3 |
| 1,2 | >300 | 196,6 | >300 | 101,2 |
| 1,4 | >300 | 195,5 | >300 | 100,4 |
| 1,6 | >300 | 200,5 | >300 | 99,7 |
| 1,8 | >300 | 201,0 | >300 | 100,2 |
| 2,0 | >300 | 201,0 | >300 | 101,6 |

### Beispiel 6

### Bestimmung des F.V-Defekts in Plasma von Patienten unter Marcumar-Therapie

Von 9 Plasmen von Patienten, die weder Marcumar noch Heparin erhielten sowie von 6 Plasmen von Patienten nach Thrombosen unter Marcumar-Therapie wurde die APCT entsprechend Beispiel 1 und die APCV mit einem F.V-MP Anteil am Probenvolumen von 75 % entsprechend Beispiel 2 bestimmt.

In Tabelle 6 sind die erhaltenen Gerinnungszeiten aufgeführt. Die Ergebnisse wurden in % relativ zu den Gerinnnungszeiten die mit SHP erhalten wurden (= 100 %) umgerechnet. Positiv sind in beiden Tests solche Proben, die unterhalb der 100 %-Grenze liegen. Die nicht-marcumarisierten Plasmen wurden in beiden Tests positiv, d.h. unterhalb der 100 %-Grenze gefunden, während alle marcuma-' risierten Plasmen in der APCT negativ, im Verfahren (APCV) jedoch 5 von 6 Plasmen deutlich unterhalb dieser Grenze lagen. Durch die Mischung mit dem F.V-MP werden in den marcumarisierten Plasmen alle defekten, Vitamin-K-abhängigen Enzyme substituiert. Auch bei Mischung der Plasmen mit normalem Plasma (Tabelle 6) werden die Gerinnungszeiten verkürzt. Dies genügt jedoch nicht, um mit derart substituierten Proben in der APCT positive Proben zu identifizieren.

**Tabelle 6:**

| Reaktionsverhalten von Plasmen mit F.V-Leiden Defekt und von marcumarisierten Plasmen von Patienten nach Thrombosen in der APCT und der APCV. Angabe der Gerinnungszeiten relativ zu SHP (= 100) in %. F.V-L = Plasmen mit homozygoten F.V-Leiden Defekt, Marc = marcumarisierte Plasmen, Marc/SHP = marcumarisierte Plasmen 1+1 gemischt mit normalem, nicht-marcumarisiertem Plasma. | | | | | |
|---|---|---|---|---|---|
| APCT | | | APCV | | |
| F.V-L | Marc | Marc/SHP | F.V-L | Marc | Marc/SHP |
| 60,3 | 196,6 | 99,9 | 60,6 | 74,6 | 85,1 |
| 46,6 | 198,5 | 100,4 | 46,6 | 74,4 | 84,4 |
| 48,9 | 219,7 | 103,8 | 51,5 | 81,2 | 90,3 |
| 42,4 | 143,9 | 96,7 | 44,0 | 82,3 | 88,0 |
| 44,6 | 245,5 | 74,7 | 46,5 | 58,6 | 104,9 |
| 56,1 | 180,0 | 110,6 | 52,8 | 105,6 | 99,2 |
| 54,3 | | | 54,4 | | |
| 49,2 | | | 51,2 | | |
| 62,4 | | | 55,9 | | |

### Beispiel 7

### Bestimmung des F.V-Defekts durch Aktivierung des Protein C im zugesetzten Faktor V-Mangelplasma

Die Bestimmung der Gerinnungszeit erfolgte an einem automatischen Koagulometer (Behring Fibrintimer A, Fa. Behringwerke; Fa. Behringwerke AG, Marburg). Alle Reagenzien waren von der Fa. Behringwerke AG.

Für die Bestimmung der Gerinnungszeit wurde 1 Abfüllung Protein-Aktivator Reagenz für Protein C-Reagenzien der Fa. Behringwerke mit 1 Abfüllung Pathromtin® SL (ein APTT-Reagenz auf Silica-Basis; 5,5 ml) gelöst. Dieses Reagenz und die Calciumchloridlösung (25 mM) wurden vor Gebrauch auf +37 °C erwärmt. Faktor V-Mangelplasma wurde in 1 ml destilliertem Wasser gelöst.

In ein Meßröhrchen wurden nacheinander pipettiert
x µl Plasmaprobe
y µl F.V-MP
100 µl Protein C-Aktivator/Pathromtin® SL-Gemisch.

Anschließend wurde bei +37 °C für 3 Minuten inkubiert und durch Zugabe von 100 µl Startreagenz die Gerinnungszeit gestartet. Die Gerinnungszeit wurde bei 405 nm bestimmt.

Die Volumina x und y wurden so gewählt, daß das Gesamtvolumen (x+y) exakt 100 pl betrug. Dabei entspricht die Variante mit 0 µl F.V-MP einem neuen Test zum Screening auf Störungen des Protein C/Protein S-Systems, wie in der deutschen Patentanmeldung P 44 27 785.7 beschrieben. Die Gerinnungszeiten wurden in den gleichen Proben wie in Beispiel 2 bestimmt.

In Tabelle 7 sind die erhaltenen Gerinnungszeiten sowie die Differenzen der Gerinnungszeiten relativ zu SHP aufgeführt. Wie in den anderen Testvarianten (Beispiele 2 bis 4) werden die Gerinnungszeiten mit steigendem Anteil von F.V-MP am Probenvolumen verlängert. Die Differenzen zwischen SHP und Protein S-Mangelplasma werden mit steigendem Anteil an F.V-MP geringer, der Effekt des F.V-Leiden hingegen größer. Bei einem F.V-MP Anteil von 70 % am Probenvolumen ist der Effekt des Protein-S Defizits bereits neutralisiert. Die Ergebnisse sind somit der unter Beispiel 4 gezeigten Anwendung des erfindungsgemäßen Verfahrens ebenbürtig, obwohl in diesem Fall nicht aktiviertes Protein C zugegeben wurde, sondern alternativ nicht-aktiviertes Protein C erst im Testansatz aktiviert wurde.

**Tabelle 7: Gerinnungszeiten verschiedener Plasmen mit Mangel- bzw. Defekten die die Funktionalität des Protein C/Protein S-Systems beeinflussen im Verfahren auf Basis einer modifizierten APTT bei Aktivierung von Protein C erst im Testansatz. Angabe der erhaltenen Gerinnungszeiten (A) bzw. deren Differenzen (B) zu SHP in Sekunden. SHP = normales Plasma; PC-MP = Protein C-Mangelplasma, PS-MP = Protein S-Mangelplasma, F.V-L1/1 = Plasma mit homozygoten F.V-Leiden Defekt, F.V-L1/2 = Plasma mit heterzygoten F.V-Leiden Defekt.**

| | | Anteil von F.V-Mangelplasma | | | |
|---|---|---|---|---|---|
| | Plasma | 50% | 60% | 70% | 75% |
| (A) | SHP | 154,9 | 170,0 | 195,0 | 207,2 |
| | PC-MP | 176,4 | 206,6 | 263,7 | 274,4 |
| | PS-MP | 122,5 | 156,0 | 196,3 | 241,2 |
| | F.V-L1/1 | 69,4 | 78,9 | 93,6 | 103,4 |
| | F.V-L1/2 | 94,7 | 104,4 | 125,7 | 131,3 |
| (B) | PC-MP | 21,5 | 36,6 | 68,7 | 67,2 |
| | PS-MP | -32,4 | -14,0 | 1,3 | 34,0 |
| | F.V-L1/1 | -85,5 | -91,1 | -101,4 | -103,8 |
| | F.V-L1/2 | -60,2 | -65,6 | -69,3 | -75,9 |

### Beispiel 8

### Dämpfung des Faktor VIII-Einflusses der Probe in einer APTT-abhängigen Methode durch Zusatz von Faktor VIII zu einem Faktor V-Mangelplasma.

Die Bestimmung der Gerinnungszeit erfolgte an einem automatischen Koagulometer (Behring Fibrintimer A, Fa. Behringwerke; Fa. Behringwerke AG, Marburg). Alle Reagenzien waren von der Fa. Behringwerke AG. Als Faktor VIII-Quelle wurde Beriate®, ein humanes Faktor VIII-Konzentrat der Fa. Behringwerke verwendet.

Die Testdurchführung erfolgte nach dem in Beispiel 2 beschriebenen Verfahren, wobei ein Mischungsverhältnis von Probe und Faktor V-Mangelplasma von 25:75 gewählt wurde. Als Faktor V-Mangelplasma wurde ein hitzeinaktiviertes humanes Plasma verwendet. Bei dieser Inaktivierung wird sowohl Faktor V, als auch Faktor VIII zerstört. Zum Vergleich wurde dieses Plasma nach Einlösen mittels Beriate® mit Faktor VIII auf eine Konzentration von 1 Einheit pro ml substituiert. Weiterhin wurde SHP mit Faktor VIII aus Beriate® substituiert, so daß der Faktor VIII-Gehalt zwischen 1 und 4 Einheiten/ml betrug. Zum Vergleich wurde ein humanes Plasma mit heterozygotem Faktor V-Leiden Defekt im erfindungsgemäßen Verfahren bestimmt.

In Tabelle 8 sind die Gerinnungszeiten sowie die Differenzen der Gerinnungszeiten relativ zu SHP (enthaltend 1E/ml Faktor VIII) aufgeführt, die mit dem Faktor V-Mangelplasma ohne bzw. mit Faktor VIII-Substitution erhalten wurden. Bei Verwendung des Faktor VIII-freien Mangelplasmas werden im normalen Plasma mit steigendem Faktor VIII-Gehalt des Plasmas die Gerinnungszeiten verkürzt und nähern sich denen an, die mit einem Plasma mit Faktor V-Leiden Defekt, aber normalem Faktor VIII-Gehalt erhalten werden. Enthält das Faktor V-Mangelplasma hingegen bereits 1 E/ml Faktor VIII, so ist die Verkürzung der Gerinnungszeit in Abhängigkeit vom Faktor VIII-Gehalt der Probe vernachlässigbar.

Dies zeigt, daß in einem auf einer APTT basierenden Testaufbau durch Zusatz von Faktor VIII bzw. bei Verwendung von Faktor Vlll-haltigen Reagenzien eine falsch positive Identifizierung von Proben auf Grund erhöhter Faktor VIII Spiegel vermieden werden kann.

**Tabelle 8: Gerinnungszeiten von normalen Plasma in Abhängigkeit vom Faktor VIII-Gehalt bei Verwendung von Faktor V-Mangelplasma ohne Faktor VIII (0 F.VIII) bzw. bei Verwendung eines Faktor V-Mangelplasmas mit 1 Einheit.Faktor VIII pro ml. Neben den Gerinnungszeiten für normales Plasma (SHP) mit verschiedenen Faktor VIII-Konzentrationen (1 bis 4 E/ml) sind die Gerinnungszeiten für Plasma mit heterozygoten F.V-Leiden Defekt (F.V-Leiden), sowie die Differenzen relativ zum normalen Plasma mit 1 E Faktor VIII/ml angegeben. Werte in Sekunden.**

| | Absolutwerte | | Differenzen | |
|---|---|---|---|---|
| **SHP** | **0 F.VIII** | **1 E/ml F.VIII** | **0 F.VIII** | **1 E/ml F.VIII** |
| **1** | 181,8 | 76,9 | 0,0 | 0,0 |
| **1,5** | 150,9 | 75,3 | -30,9 | -1,6 |
| **2** | 134,2 | 73,7 | -47,6 | -3,2 |
| **2,5** | 123,3 | 73,1 | -58,5 | -3,8 |
| **3** | 116,1 | 72,1 | -65,7 | -4,8 |
| **4** | 109,7 | 69,6 | -72,1 | -7,3 |
| **F.V-Leiden** | 85,7 | 48,4 | -96,1 | -28,5 |

## Patentansprüche

1. Verfahren zum qualitativen Nachweis und zur quantitativen Bestimmung der Stabilität des aktivierten Gerinnungsfaktors V gegenüber proteolytischem Abbau in einer Probe einer biologischen Flüssigkeit, das folgende Schritte einschließt:
a) Mischen der Probe mit Faktor V-Mangelplasma humanen oder tierischen Ursprungs als Reagenz A, dessen Gehalt an funktionsfähigem Faktor V gegenüber normalem, humanen Plasma verringert ist und das Zusätze zur Neutralisierung von Heparin enthält und Faktor VIII im Konzentrationsbereich zwischen 0,7 und 1,3 E/ml enthält,
b) Zugabe eines Reagenz B zur Aktivierung des Faktor V der Probe,
c) Zugabe eines Reagenz C zum proteolytischen Abbau des aktivierten Faktor V der Probe,
d) Zugabe von Reagenzien zur Bestimmung der residualen Faktor Va-Aktivität,
wobei der Anteil des Probenvolumens am gesamten Testvolumen maximal 20 %, vorzugsweise jedoch weniger oder gleich 10 % beträgt.

2. Verfahren nach Anspruch 1, wobei es sich bei dem Reagenz A um Faktor V-Mangelplasma humanen Ursprungs handelt.

3. Verfahren nach mindestens einem der Ansprüche 1-2, wobei der Mangel an Faktor V als ein funktioneller Mangel aufgefasst wird und damit sowohl Plasmen mit einem tatsächlich verringerten Gehalt an Faktor V verwendet werden können, als auch Plasmen, die zwar Faktor V enthalten, dessen Faktor V aber in dem angewandten Aktivierungsverfahren nicht aktivierbar ist.

4. Verfahren nach Anspruch 3, wobei der verringerte Gehalt an Faktor V im Plasma entweder auf einem genetischen Mangel oder Defekt des Spenders beruht.

5. Verfahren nach Anspruch 3, wobei der verringerte Gehalt an Faktor V im Plasma durch Adsorption an geeignete affine Bindungspartner, bevorzugterweise an mit Antikörper gegen Faktor V beschichteten Trägermaterialien, besonders bevorzugt durch Immunadsorption mittels monoklonaler Antikörper gegen Faktor V erzeugt wurde.

6. Verfahren nach Anspruch 3, wobei der Faktor V des verwendeten Plasmas durch Vorbehandlungen inaktiviert wurde, wobei besonders bevorzugt eine Hitzebehandlung zur Denaturierung des Faktor V verwendet wird.

7. Verfahren nach Anspruch 3, wobei der funktionsfähige Faktor V des verwendeten Plasmas durch Vorbehandlungen mit proteolytischen Enzymen, die spezifisch Faktor V oder Faktor Va inaktivieren, verringert wurde.

8. Verfahren nach Anspruch 3, wobei der im Plasma vorkommende Faktor V nicht durch das Reagenz B aktivierbar ist.

9. Verfahren nach Anspruch 2, wobei der aktivierbare, funktionsfähige Gehalt von Faktor V zwischen <1 bis 50 %, bevorzugterweise jedoch weniger als 10 % des Gehalts eines humanen Plasmas beträgt.

10. Verfahren nach Anspruch 1 und 2, wobei der Anteil des Faktor V-Mangelplasmas nach Mischung mit der Probe zwischen 50 - 95 % beträgt, bevorzugterweise jedoch im Bereich 60 - 80 % liegt.

11. Verfahren nach Anspruch 1 und 2, wonach das Faktor V-Mangelplasma Polybren in Konzentrationen zwischen 2 und 50 mg/l, besonders bevorzugt im Bereich zwischen 10 und 20 mg/l enthält.

12. Verfahren nach Anspruch 1, wobei es sich bei dem Reagenz, mit dem die Probe gemischt wird, um Wasser, physiologische Kochsalzlösung, eine geeignete Pufferlösung und/oder um einzelne oder mehrere Enzyme und Cofaktoren handelt, die nicht mit Faktor V identisch sind.

13. Verfahren nach Anspruch 12, wobei in dem Reagenz für die Reaktion geeignete Konzentrationen an Protein S, vorzugsweise im Konzentrationsbereich - bezogen auf die Konzentration nach Mischung mit der Probe - von 0,2 - 5 E/ml, besonders bevorzugt im Konzentrationsbereich 0,5 - 2 E/ml enthalten sind.

14. Verfahren nach Anspruch 12, wobei in dem Reagenz für die Reaktion geeignete Konzentrationen an Faktor X, vorzugsweise im Konzentrationsbereich - bezogen auf die Konzentration nach Mischung mit der Probe - von 0,2 - 5 E/ml, besonders bevorzugt im Konzentrationsbereich 0,5 - 2 E/ml enthalten sind.

15. Verfahren nach Anspruch 12, wobei in dem Reagenz für die Reaktion geeignete Konzentrationen an Prothrombin, vorzugsweise im Konzentrationsbereich - bezogen auf die Konzentration nach Mischung mit der Probe - von 0,5 -10 E/ml, besonders bevorzugt im Konzentrationsbereich 1,0 - 2 E/ml enthalten sind.

16. Verfahren nach Anspruch 12, wobei in den Reagenzien Phospholipide, bevorzugterweise Gemische von Phosphatidylcholin, Phosphatidylserin und Phosphatidylethanolamin in Konzentrationen enthalten sind, die bezogen auf die Konzentration nach Mischung mit der Probe im Bereich von 0,3 - 0,001 %, bevorzugterweise im Bereich von 0,01 - 0,1 % liegen.

17. Verfahren nach mindestens einem der Ansprüche 1 und 12-16, wobei ein Reagenz zur Mischung der Probe eines oder mehrerer der unter Anspruch 13 - 16 genannten Zusätze enthält.

18. Verfahren nach Anspruch 1, wobei die Aktivierung des Faktor V der Probe direkt oder indirekt erfolgen kann.

19. Verfahren nach Anspruch 18, wobei die Akivierung des Faktor V direkt durch Zugabe geeigneter aktivierender Enzyme humanen oder tierischen Ursprungs erfolgen kann.

20. Verfahren nach Anspruch 19, wobei als Enzym humanen Ursprungs bevorzugterweise Thrombin verwendet wird.

21. Verfahren nach Anspruch 19, wobei als Enzyme nicht-humanen Ursprungs bevorzugterweise Faktor V-Aktivatoren aus Schlangengift, besonders bevorzugt Faktor V-Aktivator aus dem Gift von Vipera russellii, verwendet wird.

22. Verfahren nach Anspruch 18, wobei die indirekte Aktivierung des Faktor V der Probe durch Auslösung der Gerinnungskaskade durch Aktivierung der Kontaktphase mittels Oberflächenaktivatoren, Phospholipiden und Calciumionen nach dem Prinzip der aktivierten, partiellen Thromboplastin-Zeit erfolgen kann.

23. Verfahren nach Anspruch 18, wobei die indirekte Aktivierung des Faktor V der Probe durch Auslösung der Gerinnungskaskade durch Zugabe von Thromboplastin, Phospholipiden und Calciumionen nach dem Prinzip der Thromboplastin-Zeit erfolgen kann.

24. Verfahren nach Anspruch 18, wobei die Aktivierung des Faktor V der Probe indirekt durch Aktivierung von Prothrombin durch Zusatz von Thrombin-aktivierenden Substanzen, beispielsweise aus dem Schlangengift von Echis carinatus erfolgen kann.

25. Verfahren nach Anspruch 1, wonach der Abbau des aktivierten Faktor V der Probe durch Zugabe eines proteolytischen Enzyms humanen oder tierischen Ursprungs erfolgt.

26. Verfahren nach Anspruch 25, wobei der Abbau des aktivierten Faktor V durch humanes Protein C erfolgt.

27. Verfahren nach Anspruch 26, wobei das humane Protein C bereits in aktivierter Form zum Testansatz gegeben wird, bevorzugterweise in Konzentration - bezogen auf den Testansatz von 0,02 -1 E/ml, besonders bevorzugterweise von 0,05 - 0,15 E/ml.

28. Verfahren nach mindestens einem der Ansprüche 2 und 26, wobei das humane Protein C in nicht-aktivierter Form zugegeben wird oder das Protein C im Faktor V Mangelplasma erst im Testansatz aktiviert wird.

29. Verfahren nach mindestens einem der Ansprüche 1 und 28, wonach ein Protein C aktivierendes Enzym in mindestens einem der Reagenzien enthalten ist, das mit der Probe und dem humanen, nicht-aktivierten Protein C in Kontakt kommt und dieses aktiviert.

30. Verfahren nach Anspruch 29, wobei bevorzugterweise zur Aktivierung von Protein C Enzyme aus dem Schlangengift der Gattung Agkistrodon verwendet werden.

31. Verfahren nach Anspruch 1, wobei die Bestimmung der residualen Faktor Va-Aktivität im Testansatz durch Bestimmung der Aktivierung von Prothrombin zu Thrombin erfolgt.

32. Verfahren nach mindestens einem der Ansprüche 1 und 31, wobei die Bestimmung der Thrombinaktivität durch Bestimmung der Umsetzung von Fibrinogen an Hand mechanischer, optischer oder opto-mechanischer Verfahren erfolgt und das Fibrinogen bevorzugterweise aus der Probe, besonders bevorzugt aber aus dem Reagenz stammt, mit dem die Probe gemischt wird.

33. Verfahren nach Anspruch 32, wobei die Bestimmung der Thrombinaktivität durch Bestimmung der Umsetzung eines thrombinspezifischen, chromogenen Substrats durch optische Detektion erfolgt.

34. Verfahren nach mindestens einem der Ansprüche 1, 12-17 und 31, wobei die Spaltung von Prothrombin durch einen Faktor Va stimulierbaren Prothrombinaktivator erfolgt.

35. Verfahren nach Anspruch 34, wobei die eingesetzten Prothrombinaktivatoren humanen und nicht-humanen Ursprungs sein können.

36. Verfahren nach Anspruch 35, wobei als humaner Prothrombinaktivator Faktor X verwendet wird.

37. Verfahren nach Anspruch 35, wobei als nicht-humane Prothrombinaktivatoren solche aus Rind verwendet werden.

38. Verfahren nach Anspruch 1, wobei die unter Anspruch 1 genannten Schritte durch Zeiträume zur Inkubation der entstandenen Gemische unterbrochen sein können, beziehungsweise innerhalb der Schritte Inkubationsphasen auftreten können.

39. Verfahren nach Anspruch 1, wobei durch Kombination der Reagenzien die Probe mit nur wenigen, bevorzugterweise nur mit 2 Reagenzien, ganz bevorzugterweise mit nur 1 Reagenz, das alle notwendigen Komponenten enthält, in Kontakt gebracht wird.

40. Verfahren nach Anspruch 35, wobei als nicht-humane Prothrombinaktivatoren solche aus Schlangengift, bevorzugt aus dem Gift von Notechis scutatus scutatus, verwendet werden.

41. Verfahren nach Anspruch 3 bis 7, wobei das nach diesen Verfahren hergestellte Faktor V Mangelplasma Faktor VIII in Konzentrationen zwischen 0,7 und 1,3 E/ml enthält bzw. durch Zusatz von gereinigtem Faktor VIII auf diese Konzentrationen substituiert wird.

42. Verfahren nach Anspruch 12, wobei in den Reagenzien Faktor VIII enthalten ist, dessen Konzentration nach Mischung mit der Probe im Bereich von 0,01 bis 2 Einheiten/ml, bevorzugterweise im Konzentrationsbereich von 0,7 bis 1,3 Einheiten/ml beträgt, wobei 1 Einheit als diejenige funktionelle Menge definiert ist, die in einem normalen, humanen Plasma in 1 ml vorhanden ist.

43. Verfahren zum qualitativen Nachweis und zur quantitativen Bestimmung der Stabilität des aktivierten Gerinnungsfaktors V gegenüber proteolytischem Abbau in einer Probe einer biologischen Flüssigkeit, das folgende Schritte einschliesst:
a) Mischen der Probe mit einem Reagenz A, dessen Gehalt an funktionsfähigem Faktor V gegenüber normalem, humanen Plasma verringert ist,
b) Zugabe eines Reagenz B zur Aktivierung des Faktor V der Probe,
c) Zugabe eines Reagenz C zum proteolytischen Abbau des aktivierten Faktor V der Probe,
d) Zugabe von Reagenzien zur Bestimmung der residualen Faktor Va-Aktivität,
wobei der Anteil des Probenvolumens am gesamten Testvolumen maximal 20 %, vorzugsweise jedoch weniger oder gleich 10 % beträgt und wobei die Aktivierung des Faktors V der Probe direkt durch Zugabe von Faktor V-Aktivatoren aus Schlangengift, besonders bevorzugt Faktor V-Aktivator aus dem Gift von Vipera russelli, erfolgt.

44. Verfahren nach Anspruch 43, wobei es sich bei dem Reagenz A um Faktor V-Mangelplasma humanen oder tierischen Ursprungs handelt.

45. Verfahren nach einem der Ansprüche 43 und 44, wobei der Abbau des aktivierten Faktor V durch humanes Protein C erfolgt, das in bereits aktivierter Form zum Testansatz gegeben wird.

46. Verfahren nach einem der Ansprüche 43 und 44, wobei das humane Protein C in nicht-aktivierter Form zugegeben wird.

## Claims

1. A method for qualitatively detecting and for quantitatively determining the stability of the activated coagulation factor V toward proteolytic degradation in a sample of a biological fluid, which method includes the following steps:
a) mixing the sample with factor V-deficient plasma of human or animal origin as reagent A whose content of functional factor V is diminished as compared with normal, human plasma and which contains additives for neutralizing heparin and contains factor VIII in the concentration range between 0.7 and 1.3 U/ml,
b) adding a reagent B for activating the factor V of the sample,
c) adding a reagent C for proteolytically degrading the activated factor V of the sample,
d) adding reagents for determining the residual factor Va activity,
where the proportion of the total test volume represented by the sample volume is at most 20 %, preferably, however, less than or equal to 10 %.

2. The method as claimed in claim 1, wherein the reagent A is factor V-deficient plasma of human origin.

3. The method as claimed in at least one of claims 1-2, wherein the deficiency in factor V is interpreted as a functional deficiency, thereby allowing the use both of plasmas having a content of factor V which is actually diminished and of plasmas which contain factor V but whose factor V cannot be activated in the activation procedure which is employed.

4. The method as claimed in claim 3, wherein the diminished content of factor V in the plasma is due either to a genetic deficiency or defect in the donor.

5. The method as claimed in claim 3, wherein the diminished content of factor V in the plasma was produced by adsorption on suitable high-affinity binding partners, preferably on support materials coated with antibody against factor V, particularly preferably by immunoadsorption using monoclonal antibodies against factor V.

6. The method as claimed in claim 3, wherein the factor V of the plasma employed was inactivated by prior treatment, with a heat treatment particularly preferably being used to denature the factor V.

7. The method as claimed in claim 3, wherein the functional factor V of the plasma employed was diminished by prior treatment with proteolytic enzymes which specifically inactivate factor V or factor Va.

8. The method as claimed in claim 3, wherein the factor V which is present in the plasma cannot be activated by the reagent B.

9. The method as claimed in claim 2, wherein the activatable, functional content of factor V is between <1 and 50 %, preferably, however, less than 10 % of the content of a human plasma,

10. The method as claimed in claims 1 and 2, wherein the proportion of the factor V-deficient plasma after mixing with the sample is between 50-95 %, prefer-ably, however, in the range 60-80 %.

11. The method as claimed in claims 1 and 2, wherein the factor V-deficient plasma contains Polybrene at concentrations of between 2 and 50 mg/l, particularly preferably in the range between 10 and 20 mg/l.

12. The method as claimed in claim 1, wherein the reagent with which the sample is mixed is water, physiological sodium chloride solution, a suitable buffer solution and/or an individual enzyme or cofactor or a mixture of these enzymes and cofactors, which are not identical to factor V.

13. The method as claimed in claim 12, wherein the reagent for the reaction contains suitable concentrations of protein S, preferably in the concentration range - based on the concentration after mixing with the sample - of 0.2-5 U/ml, particularly preferably in the concentration range of 0.5-2 U/ml.

14. The method as claimed in claim 12, wherein the reagent for the reaction contains suitable concentrations of factor X, preferably in the concentration range - based on the concentration after mixing with the sample - of 0.2-5 U/ml, particularly preferably in the concentration range of 0.5-2 U/ml.

15. The method as claimed in claim 12, wherein the reagent for the reaction contains suitable concentrations of prothrombin, preferably in the concentration range - based on the concentration after mixing with the sample - of 0.5-10 U/ml, particularly preferably in the concentration range of 1.0-2 U/ml.

16. The method as claimed in claim 12, wherein the reagents contain phospholipids, preferably mixtures of phosphatidylcholine, phosphatidylserine and phosphatidylethanolamine at concentrations which, based on the concentration after mixing with the sample, are in the range of 0.3-0.001 %, preferably in the range of 0.01-0.1 %.

17. The method as claimed in at least one of claims 1 and 12-16, wherein a reagent for mixing with the sample contains one or more of the additives specified in claims 13-16.

18. The method as claimed in claim 1, wherein the factor V of the sample can be activated directly or indirectly.

19. The method as claimed in claim 18, wherein the factor V can be activated directly by adding suitable activating enzymes of human or animal origin.

20. The method as claimed in claim 19, wherein thrombin is preferably used as the enzyme of human origin.

21. The method as claimed in claim 19, wherein factor v activators from snake venom, particularly preferably factor V activator from the venom of Vipera russellii, are preferably used as enzymes of non-human origin.

22. The method as claimed in claim 18, wherein the factor V of the sample can be indirectly activated by triggering the coagulation cascade by means of activating the contact phase using surface activators, phospholipids and calcium ions in accordance with the principle of the activated, partial thromboplastin time.

23. The method as claimed in claim 18, wherein the factor V of the sample can be indirectly activated by triggering the coagulation cascade by means of adding thromboplastin, phospholipids and calcium ions in accordance with the principle of the thromboplastin time.

24. The method as claimed in claim 18, wherein the factor V of the sample can be indirectly activated by means of activating prothrombin by adding thrombin-activating substances, obtained, for example, from the venom of the snake Echis carinatus.

25. The method as claimed in claim 1, wherein the activated factor V of the sample is degraded by adding a proteolytic enzyme of human or animal origin.

26. The method as claimed in claim 25, wherein the activated factor V is degraded by human protein C.

27. The method as claimed in claim 26, wherein the human protein C is added to the test mixture when it is already in activated form, preferably at a concentration - based on the test mixture - of 0.02-1 U/ml, particularly preferably of 0.05-0.15 U/ml.

28. The method as claimed in at least one of claims 2 and 26, wherein the human protein C is added in non-activated form or the protein C in the factor V-deficient plasma is first activated in the test mixture.

29. The method as claimed in at least one of claims 1 and 28, wherein at least one of the reagents contains a protein C-activating enzyme which comes into contact with the sample and the human, non-activated protein C and activates the latter.

30. The method as claimed in claim 29, wherein enzymes from the venom of the snake genus Agkistrodon are preferably used for activating protein C.

31. The method as claimed in claim 1, wherein the residual factor Va activity in the test mixture is determined by determining the activation of prothrombin to form thrombin.

32. The method as claimed in at least one of claims 1 and 31, wherein the thrombin activity is determined by determining the conversion of fibrinogen using mechanical, optical or optomechanical methods, and the fibrinogen preferably derives from the sample, particularly preferably, however, from the reagent with which the sample is mixed.

33. The method as claimed in claim 32, wherein the thrombin activity is determined by determining the conversion of a thrombin-specific, chromogenic substrate by means of optical detection.

34. The method as claimed in at least one of claims 1, 12-17 and 31, wherein the prothrombin is cleaved by a factor Va stimulatable prothrombin activator.

35. The method as claimed in claim 34, wherein the prothrombin activators which are employed can be of human and non-human origin.

36. The method as claimed in claim 35, wherein factor X is used as the human prothrombin activator.

37. The method as claimed in claim 35, wherein bovine prothrombin activators are used as non-human prothrombin activators.

38. The method as claimed in claim 1, wherein the steps specified in claim 1 can be interrupted by periods for incubating the resulting mixtures, or else incubation phases can occur within the steps.

39. The method as claimed in claim 1, wherein, by combining the reagents, the sample is brought into contact with only a few reagents, preferably only with 2 reagents, very preferably with only 1 reagent which contains all the necessary components.

40. The method as claimed in claim 35, wherein prothrombin activators from snake venom, preferably from the venom of Notechis scutatus scutatus, are used as the non-human prothrombin activators.

41. Any one of the methods as claimed in claims 3 to 7, wherein the factor V-deficient plasma prepared in accordance with these methods contains factor VIII at concentrations of between 0.7 and 1.3 U/ml, or is supplemented to achieve these concentrations by adding purified factor VIII.

42. The method as claimed in claim 12, wherein the reagents contain factor VIII at a concentration which, based on the concentration after mixing with the sample, is in the range of from 0.01 to 2 units/ml, preferably in the concentration range of from 0.7 to 1.3 units/ml, where 1 unit is defined as the functional quantity which is present in 1 ml of a normal, human plasma.

43. A method for qualitatively detecting and for quantitatively determining the stability of the activated coagulation factor V toward proteolytic degradation in a sample of a biological fluid, which method includes the following steps:
a) mixing the sample with a reagent A whose content of functional factor V is diminished as compared with normal, human plasma,
b) adding a reagent B for activating the factor V of the sample,
c) adding a reagent C for proteolytically degrading the activated factor V of the sample,
d) adding reagents for determining the residual factor Va activity,
where the proportion of the total test volume represented by the sample volume is at most 20 %, preferably, however, less than or equal to 10 % and where the activation of factor V of the sample is effected by addition of factor V activators from snake venom, particularly preferably factor v activator from the venom of *Vipera russellii*.

44. The method as claimed in claim 43, wherein the reagent A is factor V-deficient plasma of human or animal origin.

45. The method as claimed in either of claims 43 and 44, wherein the activated factor V is degraded by human protein C added to the test mixture when it is already in activated form.

46. The method as claimed in either of claims 43 and 44, wherein the human protein C is added in non-activated form.

## Revendications

1. Procédé pour la détermination qualitative et pour la détermination quantitative de la stabilité du facteur de coagulation V activé vis-à-vis de la dégradation protéolytique dans un échantillon d'un liquide biologique, qui comprend les étapes suivantes :
a) mélange de l'échantillon avec un plasma déficient en facteur V, d'origine humaine ou animale comme réactif A, dont la teneur en facteur V fonctionnel est réduite par rapport à un plasma humain normal, et contient l'additif pour la neutralisation de l'héparine et contient le facteur VIII à des concentrations comprises entre 0,7 et 1,3 U/ml,
b) addition d'un réactif B pour l'activation du facteur V de l'échantillon,
c) addition d'un réactif C pour la dégradation protéolytique du facteur V activé de l'échantillon,
d) addition de réactifs pour la détermination de l'activité résiduelle du facteur Va,
dans lequel la proportion du volume de l'échantillon par rapport au volume total de l'essai est de 20 % au maximum, mais est de préférence inférieure ou égale à 10 %.

2. Procédé selon la revendication 1, dans lequel, en ce qui concerne le réactif A, il s'agit de plasma déficient en facteur V, d'origine humaine.

3. Procédé selon au moins l'une des revendications 1-2, dans lequel la déficience en facteur V est considérée comme une déficience fonctionnelle et où on peut par conséquent utiliser aussi bien des plasmas ayant une teneur effectivement réduite en facteur V que des plasmas qui contiennent certes du facteur V, mais dont le facteur V n'est pas activable dans le procédé d'activation utilisé.

4. Procédé selon la revendication 3, dans lequel la teneur réduite en facteur V du plasma repose sur une déficience ou une anomalie génétique du donneur.

5. Procédé selon la revendication 3, dans lequel la teneur réduite en facteur V du plasma a été engendrée par adsorption sur des partenaires de liaison d'affinité appropriés, de préférence sur des matériaux de support tapissés d'anticorps dirigés contre le facteur V, de façon particulièrement préférée par immuno-adsorption à l'aide d'anticorps monoclonaux dirigés contre le facteur V.

6. Procédé selon la revendication 3, dans lequel le facteur V du plasma utilisé a été inactivé par des prétraitements, un traitement par la chaleur étant utilisé de façon particulièrement préférée pour la dénaturation du facteur V.

7. Procédé selon la revendication 3, dans lequel la teneur en facteur V fonctionnel du plasma utilisé a été réduite par des prétraitements par des enzymes protéolytiques qui inactivent spécifiquement le facteur V ou le facteur Va.

8. Procédé selon la revendication 3, dans lequel le facteur V présent dans le plasma n'est pas activable par le réactif B.

9. Procédé selon la revendication 2, dans lequel la teneur fonctionnelle activable en facteur V est comprise entre < 1 et 50 %, mais de préférence est inférieure à 10 % de la teneur d'un plasma humain.

10. Procédé selon les revendications 1 et 2, dans lequel la proportion du plasma déficient en facteur V, après mélange avec l'échantillon, est comprise entre 50 et 95 %, mais se situe de préférence dans la plage de 60-80 %.

11. Procédé selon les revendications 1 et 2, selon lequel le plasma déficient en facteur V contient du Polybren à des concentrations comprises entre 2 et 50 mg/ml, de façon particulièrement préférée dans la plage comprise entre 10 et 20 mg/ml.

12. Procédé selon la revendication 1, dans lequel, en ce qui concerne le réactif avec lequel est mélangé l'échantillon, il s'agit d'eau, d'une solution physiologique de chlorure de sodium, d'une solution tampon appropriée et/ou d'une ou plusieurs enzymes et d'un ou plusieurs cofacteurs, qui ne sont pas identiques au facteur V.

13. Procédé selon la revendication 12, dans lequel, dans le réactif pour la réaction sont contenues des concentrations appropriées de protéine S, de préférence dans la plage de concentrations - par rapport à la concentration après mélange avec l'échantillon - de 0,2-5 U/ml, de façon particulièrement préférée dans la plage de concentrations de 0,5-2 U/ml.

14. Procédé selon la revendication 12, dans lequel, dans le réactif pour la réaction, sont contenues des concentrations appropriées de facteur X, de préférence dans la plage de concentrations - par rapport à la concentration après mélange avec l'échantillon - de 0,2-5 U/ml, de façon particulièrement préférée dans la plage de concentrations de 0,5-2 U/ml.

15. Procédé selon la revendication 12, dans lequel, dans le réactif pour la réaction, sont contenues des concentrations appropriées de prothrombine, de préférence dans la plage de concentrations - par rapport à la concentration après mélange avec l'échantillon - de 0,5-10 U/ml, de façon particulièrement préférée dans la plage de concentrations de 1,0-2 U/ml.

16. Procédé selon la revendication 12, dans lequel, dans les réactifs sont contenus des phospholipides, de préférence des mélanges de phosphatidylcholine, phosphatidylsérine et phosphatidyléthanol-amine, à des concentrations qui, par rapport à la concentration après mélange avec l'échantillon, se situent dans la plage allant de 0,001 à 0,3 %, de préférence dans la plage allant de 0,01 à 0,1 %.

17. Procédé selon au moins l'une des revendications 1 et 12 à 16, dans lequel un réactif destiné au mélange de l'échantillon contient un ou plusieurs des additifs mentionnés dans les revendications 13-16.

18. Procédé selon la revendication 1, dans lequel l'activation du facteur V de l'échantillon peut s'effectuer directement ou indirectement.

19. Procédé selon la revendication 18, dans lequel l'activation du facteur V peut s'effectuer directement par addition d'enzymes activatrices appropriées, d'origine humaine ou animale.

20. Procédé selon la revendication 19, dans lequel on utilise comme enzyme d'origine humaine de préférence de la thrombine.

21. Procédé selon la revendication 19, dans lequel on utilise comme enzyme d'origine non humaine de préférence des activateurs de facteur V provenant de venin de serpent, de façon particulièrement préférée l'activateur de facteur V provenant du venin de *Vipera russellii.*

22. Procédé selon la revendication 18, dans lequel l'activation indirecte du facteur V de l'échantillon peut s'effectuer par déclenchement de la cascade de coagulation par activation de la phase de contact au moyen d'activateurs de surface, de phospholipides et d'ions calcium, selon le principe du temps de céphaline activé.

23. Procédé selon la revendication 18, dans lequel l'activation indirecte du facteur V de l'échantillon peut s'effectuer par déclenchement de la cascade de coagulation par addition de thromboplastine, de phospholipides et d'ions calcium, selon le principe du temps de céphaline.

24. Procédé selon la revendication 18, dans lequel l'activation du facteur V de l'échantillon peut s'effectuer indirectement par activation de la prothrombine par addition de substances activant la thrombine, par exemple, provenant du venin de serpent *d'Echis carinatus.*

25. Procédé selon la revendication 1, selon lequel la dégradation du facteur V activé de l'échantillon s'effectue par addition d'une enzyme protéolytique d'origine humaine ou animale.

26. Procédé selon la revendication 25, dans lequel la dégradation du facteur V activé s'effectue au moyen de protéine C humaine.

27. Procédé selon la revendication 26, dans lequel la protéine C humaine est ajoutée déjà sous forme activée au mélange d'essai, de préférence à une concentration - par rapport au mélange d'essai - de 0,02-1 U/ml, de façon particulièrement préférée, de 0,05-0,15 U/ml.

28. Procédé selon au moins l'une des revendications 2 et 26, dans lequel la protéine C humaine est ajoutée sous forme non activée ou la protéine C dans le plasma déficient en facteur V n'est activée que dans le mélange d'essai.

29. Procédé selon au moins l'une des revendications 1 et 28, selon lequel une enzyme activant la protéine C est contenue dans au moins l'un des réactifs, qui entre en contact avec l'échantillon et la protéine C humaine non activée, et active celle-ci.

30. Procédé selon la revendication 29, dans lequel on utilise de préférence pour l'activation de la protéine C des enzymes provenant du venin d'un serpent appartenant au genre *Agkistrodon.*

31. Procédé selon la revendication 1, dans lequel la détermination de l'activité résiduelle du facteur Va s'effectue dans le mélange d'essai par détermination de l'activation de la prothrombine en thrombine.

32. Procédé selon au moins l'une des revendications 1 et 31, dans lequel la détermination de l'activité de la prothrombine s'effectue par détermination de la conversion du fibrinogène à l'aide de procédés mécaniques, optiques ou opto-mécaniques, et le fibrinogène provient de préférence de l'échantillon, mais de façon particulièrement préférée du réactif avec lequel est mélangé l'échantillon.

33. Procédé selon la revendication 32, dans lequel la détermination de l'activité de la thrombine s'effectue par détermination de la conversion d'un substrat chromogène spécifique de la thrombine, par détection optique.

34. Procédé selon au moins l'une des revendications 1, 12-17 et 31, dans lequel la coupure de la prothrombine s'effectue par un activateur de prothrombine pouvant être stimulé par le facteur Va.

35. Procédé selon la revendication 34, dans lequel les activateurs de prothrombine utilisés peuvent être d'origine humaine ou non humaine.

36. Procédé selon la revendication 35, dans lequel on utilise comme activateur de prothrombine d'origine humaine du facteur X.

37. Procédé selon la revendication 35, dans lequel on utilise comme activateurs de prothrombine d'origine non humaine ceux d'origine bovine.

38. Procédé selon la revendication 1, dans lequel les étapes indiquées dans la revendication 1 peuvent être interrompues par des espaces de temps pour l'incubation des mélanges résultants, ou des phases d'incubation peuvent apparaître dans les étapes.

39. Procédé selon la revendication 1, dans lequel l'échantillon est mis en contact, par association des réactifs, avec seulement quelques réactifs, de préférence avec seulement deux réactifs, de façon tout particulièrement préférée avec seulement un réactif, qui contient tous les composants requis.

40. Procédé selon la revendication 35, dans lequel on utilise comme activateurs de prothrombine d'origine non humaine ceux provenant de venin de serpent, de préférence du venin de *Notechis scutatus scutatus.*

41. Procédé selon les revendications 3 à 7, dans lequel le plasma déficient en facteur V préparé selon ce procédé contient du facteur VIII à des concentrations comprises entre 0,7 et 1,3 U/ml, ou est amené à ces concentrations par addition de facteur VIII purifié.

42. Procédé selon la revendication 12, dans lequel, dans les réactifs est contenu du facteur VIII dont la concentration se situe, après mélange avec l'échantillon, dans la plage allant de 0,01 à 2 unités/ml, de préférence dans la plage de concentrations allant de 0,7 à 1,3 unités/ml, une unité étant définie comme la quantité fonctionnelle qui, dans un plasma humain normal, est présente dans 1 ml.

43. Procédé pour la détermination qualitative et pour la détermination quantitative de la stabilité du facteur de coagulation V activé vis-à-vis de la dégradation protéolytique dans un échantillon d'un liquide biologique, qui comprend les étapes suivantes :
a) mélange de l'échantillon avec un réactif A dont la teneur en facteur V fonctionnel est réduite par rapport à un plasma humain normal,
b) addition d'un réactif B pour l'activation du facteur V de l'échantillon,
c) addition d'un réactif C pour la dégradation protéolytique du facteur V activé de l'échantillon,
d) addition de réactifs pour la détermination de l'activité résiduelle du facteur Va,
dans lequel la proportion du volume de l'échantillon par rapport au volume total de l'essai est de 20 % au maximum, mais est de préférence inférieure ou égale à 10 % et dans lequel l'activation du facteur V de l'échantillon a lieu directement par l'addition des activateurs de facteur V provenant de venin de serpent, de façon particulièrement préférée l'activateur de facteur V provenant du venin de *Vipera russellii.*

44. Procédé selon la revendication 43, dans lequel, en ce qui concerne le réactif A, il s'agit de plasma déficient en facteur V d'origine humaine ou animale.

45. Procédé selon l'une des revendications 43 et 44, dans lequel la dégradation du facteur V activé s'effectue au moyen de protéine C humaine, qui est ajoutée sous forme déjà activée au mélange d'essai.

46. Procédé selon l'une des revendications 43 et 44, dans lequel la protéine C humaine est ajoutée sous forme non activée.
